# EUROPÄISCHE PATENTANMELDUNG

(11) **EP 3 772 498 A1**
(43) Veröffentlichungstag der Anmeldung: **10.02.2021**
(21) Anmeldenummer: 19190778.1
(22) Anmeldetag: 08.08.2019
(51) Int. Cl.: C04B 35/486, A61C 13/083, A61K 6/818, B32B 18/00, C04B 35/488, C04B 35/64

(54) **VERFAHREN ZUR HERSTELLUNG EINES ZIRKONDIOXIDROHLINGS MIT FARB- UND TRANSLUZENZVERLAUF**

(71) Anmelder: VITA-ZAHNFABRIK H. Rauter GmbH & Co. KG, 79713 Bad Säckingen (DE)
(72) Erfinder: Gödiker, Michael, 79713 Bad Säckingen (DE); Kolb, Eva, 79713 Bad Säckingen (DE); Strasser Christian, 79713 Bad Säckingen (DE)
(74) Vertreter: dompatent von Kreisler Selting Werner - Partnerschaft von Patent- und Rechtsanwälten mbB

(57) **Zusammenfassung**

Verfahren zur Herstellung eines keramisches Formkörpers umfassend die folgenden Schritte:
a) Bereitstellen von 3 oder mehr keramischen Pulverschichten, die schichtweise übereinander angeordnet sind,
b) Verpressen der schichtweise übereinander angeordneten keramischen Pulverschichten zur Ausbildung eines Pressformkörpers und

Sintern des in Schritt b) erhaltenen Pressformkörpers zur Ausbildung eines keramischen Formkörpers, dadurch gekennzeichnet, dass die keramischen Pulverschichten jeweils eine voneinander verschiedene Zusammensetzung aufweisen, wobei jede keramische Pulverschicht eine Mischung aus mindestens 2 verschiedenen Basispulvern umfasst und die Basispulver jeweils mindestens 80 Gew.-% ZrO₂ und mindestens 0,02 Gew.-% Al₂O₃ aufweisen, wobei die Gewichtsangaben jeweils bezogen sind auf das Gesamtgewicht der Bestandteile des Basispulvers.

## Beschreibung

Die vorliegende Erfindung betrifft ein Verfahren, zur Herstellung eines keramischen Formkörpers, einen keramischen Formkörper, der nach dem erfindungsgemäßen Verfahren erhältlich ist sowie dessen Verwendung als dentale Restaurationen.

Für zahnmedizinische dentale Restaurationen sind unterschiedlichste organische Polymermaterialien als auch keramische Materialien bekannt. Keramische Materialien weisen regelmäßig eine höhere Festigkeit auf, sind aber für dentale Restaurationen anspruchsvoller zu bearbeiten, wenn es um deren passgenaue Herstellung geht. Für keramische dentale Restaurationen haben sich am Markt sowohl glaskeramische als auch oxidkeramische Werkstoffe etabliert. Für Glaskeramiken kommen üblicherweise Schmelzverfahren zum Einsatz, während bei oxidkeramischen Materialien pulvertechnologische Press- und Sinterverfahren erforderlich sind.

Im Stand der Technik sind mehrschichtige Blöcke für die dentale CAD-CAM Anwendung aus Feldspat- oder Leuzitkeramik bekannt. Diese entsprechen unter ästhetischen Gesichtspunkten dem Erscheinungsbild natürlicher Zähne, weisen aber in der Regel eine Festigkeit auf die im Bereich von 150 - 200 MPa liegt. Diese Festigkeiten sind jedoch insbesondere dann, wenn es sich um dentale Restaurationen mit dünnen Wandstärken handelt, weniger geeignet. Andererseits können mit geschichteten Zirkondioxidblöcken hohe Festigkeiten erzielt werden. Diese sind jedoch regelmäßig zu opak um als monolithische dentale Versorgung eingesetzt werden zu können. Der Einsatz hochfester Zirkoniumdioxid-Restaurationen erfordert somit eine manuelle Nachbearbeitung. Diese kann darin bestehen, dass die porösen Gerüste bereits vor dem Sintern mit Farbflüssigkeiten infiltriert werden, oder die gesinterten Versorgungen mit Malfarben oder Verblendkeramik individualisiert farblich der natürlichen Zahnfarbe angepasst werden.

Eine besondere Herausforderung dentaler Restaurationen liegt darin einen natürlich erscheinenden Farbverlauf in der keramischen Restauration zu erzeugen. Gleichzeitig werden an dentale Restaurationen hohe Anforderungen hinsichtlich ihrer Festigkeit, insbesondere ihrer Kantenfestigkeit, ihrer Transluzenz und Bearbeitbarkeit gestellt.

Überraschend wurde gefunden, dass die im Stand der Technik aufgezeigten Probleme durch die vorliegende Erfindung gelöst werden können. Insbesondere wurde gefunden, dass ein wenige Basispulver umfassendes System genutzt werden kann, um einen stufenlosen Farbverlauf in keramischen Formkörpern zu erzeugen, bei denen sowohl Transluzenz als auch Festigkeit des Endprodukts individuell eingestellt werden können.

Ein Gegenstand der vorliegenden Erfindung ist ein Verfahren zur Herstellung eines keramischen Formkörpers, vorzugsweise einer keramischen dentalen Restauration oder einer Vorstufe einer keramischen dentalen Restauration, umfassend die folgenden Schritte:
a) Bereitstellen von drei oder mehr keramischen Pulverschichten, die schichtweise übereinander angeordnet sind,
b) Verpressen der schichtweise übereinander angeordneten keramischen Pulverschichten zur Ausbildung eines Pressformkörpers und
c) Sintern des in Schritt b) erhaltenen Pressformkörpers zur Ausbildung eines keramischen Formkörpers, wobei die keramischen Pulverschichten jeweils eine voneinander verschiedene Zusammensetzung aufweisen und wobei jede keramische Pulverschicht eine Mischung aus mindestens zwei verschiedenen Basispulvern umfasst und die Basispulver jeweils mindestens 80 Gew.-% ZrO₂ und mindestens 0,02 Gew.-% Al₂O₃ aufweisen, wobei die Gewichtsangaben jeweils bezogen sind auf das Gesamtgewicht der Bestandteile des Basispulvers.

Im Rahmen der vorliegenden Erfindung besteht eine keramische Pulverschicht aus wenigstens zwei voneinander unterscheidbaren Basispulvern. Insbesondere bestehen die Pulverschichten aus wenigstens 2, vorzugsweise 3 oder 4, unterscheidbaren Basispulvern, wobei die keramische Pulverschicht bevorzugt als eine homogene Mischung der Basispulver vorliegt. Die keramischen Pulverschichten werden schichtweise übereinander angeordnet, wobei sich die jeweils angrenzenden Pulverschichten hinsichtlich ihrer chemischen Zusammensetzung und/oder ihrer physikalischen Eigenschaften unterscheiden. Die Unterschiede in den Zusammensetzungen der einzelnen Pulverschichten können durch die Wahl und Menge geeigneter Basispulver erfolgen. Die keramischen Pulverschichten umfassen daher mindestens zwei verschiedene Basispulver. In einer Ausführungsform umfassen wenigstens zwei, bevorzugt wenigstens drei und insbesondere alle keramischen Pulverschichten die gleichen Basispulver, jedoch in unterschiedlichen Mengen. Es hat sich gezeigt, dass mit einer begrenzten Anzahl von geeigneten Basispulvern ein Baukastensystem aufgebaut werden kann, mit denen die Eigenschaften, insbesondere die Farbe, die Transluzenz und physikalischen Eigenschaften jeder einzelnen keramischen Pulverschicht eingestellt werden kann. Neben den keramischen Bestandteilen weisen die keramischen Pulverschichten üblicherweise auch organische Bestandteile, wie etwa Presshilfsmittel, auf. Der Anteil ist jedoch, falls vorhanden, begrenzt und sollte 10 Gew.-%, bezogen auf die keramische Pulverschicht nicht übersteigen.

In einer bevorzugten Ausgestaltung weist eine oder mehrere der keramischen Pulverschichten, insbesondere jede keramische Pulverschicht, bevorzugt mindestens drei Basispulver, vorzugsweise vier Basispulver, auf.

Es hat sich als vorteilhaft herausgestellt, dass vier oder fünf keramische Pulverschichten bereitgestellt werden, die sich jeweils hinsichtlich ihrer Zusammensetzung unterscheiden. Insbesondere bei 4, speziell bei 5 oder mehr als 5 keramischen Pulverschichten lässt sich ein guter Farbübergang und Eigenschaftsübergang bewerkstelligen, der für dentale Restaurationen wichtig ist. So können hier auch insbesondere die in tieferen und dunkleren Farben ausgestalteten künstlichen Zahnhälse fließend zu den helleren Schneide- und Dentinbereichen künstlicher Zähne eingestellt werden und so den ästhetischen und mechanischen Anforderungen Rechnung getragen werden.

In einer speziell bevorzugten Ausführungsform werden in Schritt a) des erfindungsgemäßen Verfahrens fünf Pulverschichten bereitgestellt, wobei jede Pulverschicht vier voneinander unterschiedliche Basispulver aufweist, wobei jedoch in jeder Pulverschicht unterschiedliche Mengen der jeweiligen Basispulver vorliegen. Es hat sich überraschend gezeigt, dass besonders effektiv und kostengünstig gearbeitet werden kann, wenn jede keramische Pulverschicht 4 oder mehr Basispulver aufweist. Ein weiterer Gegenstand der vorliegenden Erfindung ist daher auch eine keramische Pulverschicht, die 4 oder mehr Basispulver umfasst.

In einer bevorzugten Ausgestaltung der vorliegenden Erfindung weist jede keramische Pulverschicht des Pressformkörpers ein oder mehrere färbende Metalloxide auf. In einer weiteren Ausführungsform unterscheidet sich die Konzentration der färbenden Metalloxide in jeder Pulverschicht. Bevorzugt ist jede Zwischenschicht, also jede Pulverschicht, die durch 2 direkt angrenzende Pulverschichten benachbart ist (Nachbarschichten), von einer Nachbarschicht umgeben, die eine höhere Konzentration an färbenden Metalloxiden aufweist als die Zwischenschicht. Bevorzugt ist jede Zwischenschicht von einer Nachbarschicht umgeben, die eine geringere Konzentration an färbenden Metalloxiden aufweist. Besonders bevorzugt ist jede Zwischenschicht von einer Nachbarschicht umgeben, die eine geringere Konzentration an färbenden Metalloxiden aufweist und einer Nachbarschicht, die eine höhere Konzentration an färbenden Metalloxiden aufweist.

In einer bevorzugten Ausgestaltung der Erfindung weist der Pressformkörper Pulverschichten auf, bei der von einer äußeren Pulverschicht ausgehend die Konzentration eines oder mehrerer färbender Metalloxide schichtweise zunimmt. Dies hat insbesondere den Vorteil, dass sich ein fließender Farbverlauf einstellen kann. In einer weiteren bevorzugten Ausgestaltung der Erfindung weisen eine oder mehrere der keramischen Pulverschichten des Pressformkörpers, vorzugsweise alle Pulverschichten, färbende Metalloxide in einer Menge von 0,1 bis 2,5 Gew.-%, besonders bevorzugt von 0,2 bis 2,2 Gew.-% und speziell von 0,2bis 1,5 Gew.-%, jeweils bezogen auf das Gesamtgewicht der Pulverschicht, auf.

In einer bevorzugten Ausführungsform weist der Pressformkörper Pulverschichten auf, bei denen von einer äußeren Pulverschicht ausgehend die Konzentration wenigstens eines färbenden Metalloxids, vorzugsweise bis zur gegenüberliegenden äußeren Schicht, zunimmt.

In einer bevorzugten Ausgestaltung der vorliegenden Erfindung weist jede keramische Pulverschicht des Pressformkörpers Fe₂O₃ auf. In einer weiteren Ausführungsform unterscheidet sich die Konzentration an Fe₂O₃ in jeder Pulverschicht. Bevorzugt ist jede Zwischenschicht, also jede Pulverschicht, die durch 2 direkt angrenzende Pulverschichten benachbart ist (Nachbarschichten), von einer Nachbarschicht umgeben, die eine höhere Konzentration an Fe₂O₃ aufweist als die Zwischenschicht. Bevorzugt ist jede Zwischenschicht von einer Nachbarschicht umgeben, die eine geringere Konzentration an Fe₂O₃ aufweist. Besonders bevorzugt ist jede Zwischenschicht von einer Nachbarschicht umgeben, die eine geringere Konzentration an Fe₂O₃ aufweist und einer Nachbarschicht, die eine höhere Konzentration an Fe₂O₃ aufweist.

In einer bevorzugten Ausgestaltung der Erfindung weist der Pressformkörper Pulverschichten auf, bei der von einer äußeren Pulverschicht ausgehend die Konzentration an Fe₂O₃ schichtweise zunimmt. Dies hat insbesondere den Vorteil, dass sich ein fließender Farbverlauf einstellen kann. In einer weiteren bevorzugten Ausgestaltung der Erfindung weisen eine oder mehrere der keramischen Pulverschichten des Pressformkörpers, vorzugsweise alle Pulverschichten, Fe₂O₃ in einer Menge von 0,01 bis 0,25 Gew.-%, besonders bevorzugt von 0,02 bis 0,2 Gew.-% und speziell von 0, 1 bis 0,18 Gew.-%, jeweils bezogen auf das Gesamtgewicht der Pulverschicht, auf.

In einer bevorzugten Ausgestaltung der vorliegenden Erfindung weist jede keramische Pulverschicht des Pressformkörpers Er₂O₃ auf. In einer weiteren Ausführungsform unterscheidet sich die Konzentration an Er₂O₃ in jeder Pulverschicht. Bevorzugt ist jede Zwischenschicht, also jede Pulverschicht, die durch 2 direkt angrenzende Pulverschichten benachbart ist (Nachbarschichten), von einer Nachbarschicht umgeben, die eine höhere Konzentration an Er₂O₃ aufweist als die Zwischenschicht. Bevorzugt ist jede Zwischenschicht von einer Nachbarschicht umgeben, die eine geringere Konzentration an Er₂O₃ aufweist. Besonders bevorzugt ist jede Zwischenschicht von einer Nachbarschicht umgeben, die eine geringere Konzentration an Er₂O₃ aufweist und einer Nachbarschicht, die eine höhere Konzentration an Er₂O₃ aufweist.

In einer bevorzugten Ausgestaltung der Erfindung weist der Pressformkörper Pulverschichten auf, bei der von einer äußeren Pulverschicht ausgehend die Konzentration an Er₂O₃ schichtweise zunimmt. Dies hat insbesondere den Vorteil, dass sich ein fließender Farbverlauf einstellen kann. In einer weiteren bevorzugten Ausgestaltung der Erfindung weisen eine oder mehrere der keramischen Pulverschichten des Pressformkörpers, vorzugsweise alle Pulverschichten, Er₂O₃ in einer Menge von 0,01 bis 1,5 Gew.-%, besonders bevorzugt von 0,05 bis 1,2 Gew.-% und speziell von 0,1 bis 0,9 Gew.-%, oder 0,2 bis 0,5 Gew.-%, jeweils bezogen auf das Gesamtgewicht der Pulverschicht, auf.

In einer weiteren bevorzugten Ausgestaltung weisen ein oder mehrere der Pulverschichten, vorzugsweise jede Pulverschicht, des Pressformkörpers Co₃O₄ auf. Üblicherweise kann die Menge an CO₃O₄ im Bereich von 0,001 bis 0,01, besonders bevorzugt von 0,002 bis 0,08 Gew.-% und speziell von 0,003 bis 0,006 Gew.-%, jeweils bezogen auf das Gesamtgewicht der Pulverschicht, liegen.

Die Basispulver der vorliegenden Erfindung umfassen jeweils mindestens 80 Gew.-% ZrO₂ und mindestens 0,02 Gew.-% Al₂O₃, wobei die Gewichtsangaben jeweils bezogen sind auf das Gesamtgewicht der Bestandteile des Basispulvers. In einer Ausgestaltung der vorliegenden Erfindung umfassen die Basispulver Al₂O₃ in einer Menge von 0,02 bis 0,6 Gew.-%, besonders bevorzugt 0,03 bis 0,4 Gew.-% und speziell 0,04 bis 0,2 oder 0,003 bis 0,1 oder 0,02 bis 0,08 Gew.-%, jeweils bezogen auf das Gesamtgewicht der Basispulver. Die Basispulver sind geeignet für die Herstellung dentaler Restaurationen und weisen daher auch im endgesinterten Zustand die dafür erforderlichen Anforderungen der Biokompatibilität auf. Der hohe Anteil an Zirkoniumdioxid, welches bevorzugt durch Yttriumoxid stabilisiert wird, sorgt zudem für eine hohe Festigkeit der endgesinterten Keramiken. Die Basispulver werden so gewählt, dass sie aufeinander hinsichtlich ihrer Korngrößen und ihres Sinterverhaltens aufeinander abgestimmt sind, so dass es bei der Sinterung nicht zu Sinterfehlstellen kommt. Durch das Vermischen der Basispulver kann in jeder keramischen Pulverschicht eine individuelle Einfärbung und Transluzenz erzielt werden, die wiederum so gewählt ist, dass sie mit den angrenzenden Pulverschichten, sofern vorhanden, zu einem kontinuierlichen und stufenlosen Farbverlauf führt.

Zur Phasenstabilisierung der Zirkoniumdioxid-Keramiken im gesinterten Zustand ist das Vorhandensein von Yttriumoxid oder Erbiumoxid vorteilhaft.

In einer bevorzugten Ausführungsform umfasst wenigstens ein, vorzugsweise wenigstens zwei oder drei der Basispulver, insbesondere alle Basispulver Yttriumoxid (Y₂O₃) und/oder Erbiumoxid (Er₂O₃), vorzugsweise in einer Menge von wenigstens 3 Gew.-%, insbesondere von wenigstens 5 Gew.-% oder wenigstens 6 Gew.-% und insbesondere von 4,5 - 11 Gew.-%, speziell von 6 - 10 Gew.-%, jeweils bezogen auf das Gesamtgewicht der Bestandteile des Basispulvers.

In einer Ausgestaltung der vorliegenden Erfindung weist wenigstens eins der Basispulver, vorzugsweise wenigstens zwei oder wenigstens drei der Basispulver färbende Metalloxide auf. Beispielsweise können diese färbenden Metalloxide ausgewählt sein aus der Gruppe bestehend aus Eisenoxid (Fe₂O₃), Kobaltoxid (Co₃O₄) und Erbiumoxid (Er₂O₃). Durch Zusatz der färbenden Metalloxide lassen sich individuelle Zahnfarben erstellen. Durch Vermischen mehrerer Basispulver in jeder keramischen Pulverschicht kann ein definiert abgestimmtes Material erhalten werden.

In einer Ausgestaltung der vorliegenden Erfindung weist wenigstens eins der Basispulver, vorzugsweise wenigstens zwei oder wenigstens drei der Basispulver Zirkondioxid, gegebenenfalls zusammen mit Hafniumdioxid, in einer Menge von wenigstens 89 Gew.-%, vorzugsweise in einer Menge von 89 - 98 Gew.-%, insbesondere von 90 - 96 Gew.-%, jeweils bezogen auf das Gesamtgewicht der Bestandteile des Basispulvers, auf.

In einer Ausführungsform können die Basispulver Zirkoniumdioxid und Hafniumdioxid, vorzugsweise in einem Gewichtsverhältnis von ZrO₂ zu HfO₂ von 25:1 bis 98:1, insbesondere 30:1 bis 90:1 und speziell 50:1 bis 90:1 aufweisen.

In einer bevorzugten Ausgestaltung enthalten die Pulverschichten ein Basispulver A, welches 92 - 96 Gew.-% Zirkoniumdioxid, 0,02 - 0,1 Gew.-% Aluminiumoxid, 3,5 - 6,5 Gew.-%, oder 5,0 bis 9,5 Gew.-% Yttriumoxid und 0,02 - 0,1 Gew.-% Kobaltoxid aufweist, wobei die Gewichtsangaben jeweils bezogen sind auf das Gesamtgewicht des Basispulvers A.

In einer weiteren bevorzugten Ausgestaltung weisen die Pulverschichten ein Basispulver B auf, welches 85 - 93 Gew.-% Zirkoniumdioxid, 0,02 - 0,1 Gew.-% Aluminiumoxid und 7,5 - 11 Gew.-% Erbiumoxid enthält, wobei die Gewichtsangaben jeweils bezogen sind auf das Gesamtgewicht des Basispulvers B.

In einer weiteren Ausführungsform weisen die Pulverschichten ein Basispulver C, welches 90 - 94 Gew.-% Zirkoniumdioxid, 0,02 - 0,1 Gew.-% Aluminiumoxid und 5,5 - 8,0 Gew.-%, oder 6,5 bis 9,5 Gew.-% Yttriumoxid enthält, wobei die Gewichtsangaben jeweils bezogen sind auf das Gesamtgewicht des Basispulvers C.

In einer weiteren bevorzugten Ausführungsform der vorliegenden Erfindung weisen die Pulverschichten ein Basispulver D auf, welches 90 - 94 Gew.-% Zirkoniumdioxid, 0,02 - 0,1 Gew.-% Aluminiumoxid, 5,5 - 8,0 Gew.-%, oder 6,5 bis 9,5 Gew.-% Yttriumoxid und 0,1 - 0,3 Gew.-% Eisenoxid aufweist, wobei die Gewichtsangaben jeweils bezogen sind auf das Gesamtgewicht des Basispulvers D.

In einer weiteren Ausgestaltung der vorliegenden Erfindung weisen wenigstens ein Basispulver, vorzugsweise alle Basispulver zusätzlich organische Bestandteile, vorzugsweise in einer Menge von 3 - 6 Gew.-%, insbesondere in einer Menge von 4 - 5 Gew.-% auf. Als organische Bestandteile kommen insbesondere Bindemittel und Presshilfsmittel in Frage, die im Entbinderungsschritt thermisch leicht entfernt werden können. Geeignete Binder für Zirkoniumdioxid-Sinterpulver sind dem Fachmann bekannt. Hierzu zählt beispielsweise Polyvinylalkohol (PVA).

Bevorzugt weisen die Basispulver eine Schüttdichte unterhalb von 1,2 g/cm³ auf.

Es hat sich als vorteilhaft herausgestellt Basispulver einzusetzen, die eine durchschnittliche Granulatgröße D₅₀ von 35 µm - 85 µm, bevorzugt von 40 µm - 80 µm und insbesondere von 50 µm - 70 µm oder 40 - 60 µm aufweisen. Gemessen werden die Granulatpulver trocken mittels Laserbeugung mittels eines Cilas Granulometer.

Üblicherweise weisen die anorganischen Bestandteile der Basispulver, also nach Entfernung der organischen Bestandteile, wie Bindemittel, etc., eine Partikelgröße Dso von 0,1 bis 1 µm, bevorzugt von 0,2 µm - 0.8 µm und insbesondere von 0,2 µm - 0,7 µm, gemessen mittels Laserbeugung, auf. Es wurde gefunden, dass die Partikelgrößen einen positiven Beitrag zur Sinterung und insbesondere für die Farbübergänge zwischen den einzelnen Pulverschichten sorgen.

In einer speziell bevorzugten Ausgestaltung der vorliegenden Erfindung werden in Schritt a) fünf keramische Pulverschichten schichtweise übereinander angeordnet. Die Anordnung der Schichten kann beispielsweise in einem zylindrischen Behälter erfolgen unter Ausbildung von Scheiben oder Disks. Üblicherweise kann nach jedem Schichtauftrag ein uniaxiales Verpressen der Pulverschichten erfolgen. Dies kann beispielsweise durch einen Pressstempel erfolgen wobei jedoch lediglich eine Vorverfestigung erfolgt. Das uniaxiale Verpressen der Schichten senkrecht zur Schichtoberfläche erfolgt bevorzugt bei einem Druck von 10 bis 20 MPa, insbesondere 12 bis 15 MPa.

Weiter bevorzugt erfolgt das Verpressen in Schritt b) zunächst uniaxial und senkrecht zur Schichtoberfläche, vorzugsweise unter Ausbildung eines vorverdichteten Pressformkörpers mit einer Dichte unterhalb 2,8 g/cm³, vorzugsweise mit einer Dichte im Bereich von 2,5 - 2,75 g/cm³, bspw. 2,65 g/cm³. Das uniaxiale Vorverdichten kann zu einer besseren und innigeren Vermischung und damit gleichmäßigerem Übergang zwischen den Schichten führen.

In einer weiteren Ausgestaltung des erfindungsgemäßen Verfahrens erfolgt das Verpressen in Schritt b) isostatisch, wobei das isostatische Verpressen vorzugsweise im Anschluss eines uniaxiales Vorverdichtens erfolgt, unter Ausbildung eines Pressformkörpers mit einer Dichte unterhalb von 3,4 g/cm³, insbesondere einer Dichte von 2,80 - 3,15 g/cm³, speziell mit einer Dichte von 2,85 - 3,10 g/cm³. Das isostatische Verpressen erfolgt bevorzugt nachdem alle Schichten des Formpresskörpers angeordnet sind. Geeignete Drucke für das isostatische Verpressen liegen dabei gewöhnlich im Bereich von 500 bis 10000 bar, bevorzugt im Bereich von 800 bis 8000 bar, beispielsweise 1000 bis 7000 bar oder 1000 bis 3000 bar.

Die Dicken der einzelnen Pulverschichten des Pressformkörpers können variieren. In einer bevorzugten Ausgestaltung unterscheiden sich wenigstens zwei der keramischen Pulverschichten hinsichtlich ihrer Dicke. Vorzugsweise weisen wenigstens zwei der keramischen Pulverschichten des Pressformkörpers einen Dickenunterschied von wenigstens 5% auf. Typischerweise können die Pressformkörper in Form zylindrischer, kreisförmiger Scheiben mit Durchmessern im Bereich von 50 bis 200 mm, beispielsweise 75 bis 150 mm vorliegen. Die Gesamtdicke der zylindrischen Scheiben kann dabei beispielsweise im Bereich von 8 bis 40 mm, vorzugsweise von 10 bis 30 mm, speziell von 13 bis 25 mm. Die Maße beziehen sich dabei auf den Pressformkörper im ungesinterten Zustand.

Hinsichtlich der Farbgestaltung und der anschließenden Bearbeitung hat es sich als vorteilhaft herausgestellt, dass wenigstens eine der äußeren keramischen Pulverschichten, vorzugsweise beide äußeren keramischen Pulverschichten des Pressformkörpers eine größere Dicke aufweist/aufweisen als eine zwischen den äußeren keramischen Pulverschichten liegende keramische Pulverschicht. Insbesondere bei der Verwendung der erfindungsgemäß hergestellten keramischen Formkörper für die Herstellung dentaler Restaurationen hat sich der zuvor beschriebene Schichtaufbau mit wenigstens einer dickeren äußeren Schicht als vorteilhaft erwiesen, da dies für die Bearbeitung in CAD/CAM Systemen oder anderen subtraktiven Bearbeitungsverfahren einen geeigneten Aufbau darstellt.

In einer speziell bevorzugten Ausgestaltung der vorliegenden Erfindung werden in Schritt a) des erfindungsgemäßen Verfahrens fünf keramische Pulverschichten bereitgestellt, wobei die erste Pulverschicht 20 - 30%, vorzugsweise 22 - 28%, die zweite Pulverschicht 10 - 20%, vorzugsweise 12 - 18%, die dritte Pulverschicht 15 - 25%, vorzugsweise 17 - 23%, die vierte Pulverschicht 10 - 20%, vorzugsweise 12 -18% und die fünfte Pulverschicht 20 - 30%, vorzugsweise 22 - 28% der Gesamtdicke der übereinander angeordneten Pulverschichten ausmacht und mit der Maßgabe, dass sich die Gesamtdicke auf 100% ergänzt.

In einer weiteren Ausgestaltung der vorliegenden Erfindung erfolgt die Sinterung in Schritt c) des erfindungsgemäßen Verfahrens bei einer Temperatur im Bereich von 950 - 1100°C, vorzugsweise von 980 - 1050°C unter Ausbildung eines vorgesinterten keramischen Formkörpers (Weißling). Üblicherweise erfolgt die Sinterung über einen Zeitraum, der ausreicht um die vorhandenen Bindemittel zu entfernen und dem Pressformkörper eine genügende Festigkeit für die Bearbeitung durch subtraktive Verfahren zu verleihen. Die vorgesinterten und entbinderten Pressformkörper werden als Weißlinge bezeichnet.

In einer Ausgestaltung erfolgt die Sinterung in Schritt c) des erfindungsgemäßen Verfahrens zur Herstellung des Weißlings über einen Zeitraum oberhalb von 30 Minuten, bevorzugt oberhalb von 1 Stunde, insbesondere oberhalb von 20 Stunden oder oberhalb von 50 Stunden, beispielsweise 60 bis 200 Stunden oder 70 bis 150 Stunden.

Insbesondere zur Herstellung keramischer dentaler Restaurationen bietet es sich an, dass der in Schritt c) vorgesinterte keramische Formkörper durch subtraktive Verfahren bearbeitet wird und vorzugsweise anschließend in einem weiteren Schritt endgesintert wird. Bei Anwendung subtraktiver Verfahren wird üblicherweise die Sinterschwindung mit einberechnet.

Die Endsinterung erfolgt üblicherweise bei Temperaturen oberhalb von 1350 °C, bevorzugt oberhalb von 1400 °C, speziell im Bereich von 1420 °C bis 1600 °C oder 1450 °C bis 1550°C.

Die Sinterdauer für die Endsinterung erfolgt üblicherweise über einen Zeitraum von mehr als 4 Minuten, bevorzugt mehr als 5 Minuten, insbesondere im Bereich von 5 bis 120 Minuten.

Ein weiterer Gegenstand der vorliegenden Erfindung ist ein keramischer Formkörper erhältlich nach dem erfindungsgemäßen Verfahren.

Die keramischen Formkörper können insbesondere im Dentalbereich eingesetzt werden. Hier zeichnen sie sich durch eine hohe Kantenfestigkeit bei dentalen Restaurationen, ein hervorragendes Gefüge und eine hohe 3-Punktbiegefestigkeit aus. Die keramischen Formkörper der vorliegenden Erfindung sind daher bevorzugt dentale Restaurationen, wie beispielsweise Inlays, Onlays, Kronen, Brücken, Veneers und Verblendungen oder Abutments für Implantate.

Ein weiterer Gegenstand der vorliegenden Erfindung ist die Verwendung des erfindungsgemäßen keramischen Formkörpers für dentale Restaurationen oder für die Herstellung dentaler Restaurationen.

### Beispiele:

Tabelle 1 zeigt 4 Basispulver A bis D, die für die Zusammensetzungen der keramischen Pulverschichten zum Einsatz kommen. Die Granulatgröße Dso der Basispulver liegt im Bereich von 40-80 µm. Die anorganischen Bestandteile der Basispulver weisen eine Partikelgröße Dso von 0,2 bis 0,7 µm auf.

Die Gewichtsangaben beziehen sich jeweils auf das Gesamtgewicht der Pulverzusammensetzung.

**Tabelle 1**

| **Bezeichnung** | **Komponente** | **Anteil (Gew.-%)** |
|---|---|---|
| Basispulver A | Y₂O₃ | 5,33 |
| | Al₂O₃ | 0,05 |
| | Organischer Binder | 4 |
| | Co₃O₄ | 0,05 |
| | ZrO₂ | ad 100 |
| | | |
| Basispulver B | Er₂O₃ | 9,2 |
| | Al₂O₃ | 0,045 |
| | Organischer Binder | 4 |
| | ZrO₂ | ad 100 |
| | | |
| Basispulver C | Y₂O₃ | 6,93 |
| | Al₂O₃ | 0,05 |
| | Organischer Binder | 4 |
| | ZrO₂ | ad 100 |
| | | |
| Basispulver D | Y₂O₃ | 6,09 |
| | Al₂O₃ | 0,049 |
| | Organischer Binder | 4 |
| | Fe₂O₃ | 0,2 |
| | ZrO₂ | ad 100 |

Die in der folgenden Tabelle 2 aufgeführten Anordnungen der Schichten zeigen die Zusammensetzung jeder einzelnen keramischen Pulverschicht in dem Pressformkörper. Die Pressformkörper sind für die Verwendung in der Herstellung dentaler Restaurationen vorgesehen, so dass die Schichtzusammensetzungen entsprechend der Position im Zahn ausgestaltet sind. Die Zusammensetzungen der Pulverschichten werden aus den Basispulvern gebildet, in dem die Anteile variiert werden um einen idealen Farbverlauf zu erhalten. Die Zusammensetzung jeder Pulverschicht wird durch homogenes Vermischen der Basispulver in den angegebenen Mengen erzielt. Anschließend werden die Pulver schichtweise in eine zylinderförmige Pressform mit einem Durchmesser von 100 mm gegeben und eine Schichtdicke von 18 mm eingestellt. Die Pulverschichten werden uniaxial bei einem Druck von 13 MPa senkrecht zur Schichtoberfläche vorgepresst und anschließend isostatisch bei einem Druck von 2000 bar verpresst.

Nachfolgend erfolgt die Entbinderung bei ca. 1000 °C über einen Zeitraum von ca. 100 Stunden. Die so erhaltenen Weißlinge werden über CAD/CAD Systeme zu dentalen Restaurationen gefräst.

Diese vorgesinterten und bearbeiteten Weißlinge werden anschließend bei 1450 °C über einen Zeitraum von 120 Minuten endgesintert.

**Tabelle 2**

| **Pulverschicht** | Bereich der Restauration | Basispulver C (Gew.-%) | Basispulver D (Gew.-%) | Basispulver B (Gew.-%) | Basispulver A (Gew.-%) |
|---|---|---|---|---|---|
| 1 | Schneide | 36,90 | 51,00 | 6,10 | 6,00 |
| 2 | Dentin/Schneide | 30,30 | 58,00 | 6,20 | 5,50 |
| 3 | Dentin | 17,40 | 72,00 | 6,80 | 3,80 |
| 4 | Dentin/Hals | 10,60 | 76,00 | 7,40 | 6,00 |
| 5 | Hals | 3,70 | 79,00 | 8,30 | 9,00 |

Die keramischen Pulverschichten sind im vorliegenden Beispiel so angeordnet, dass Schicht 1 (Schneide) 25%, Schicht 2 (Dentin/Schneide) 15%, Schicht 3 (Dentin) 20%, Schicht 4 (Dentin/Hals) 15% und Schicht 5 (Hals) 25% der Gesamtdicke des Pressformkörpers ausmacht.

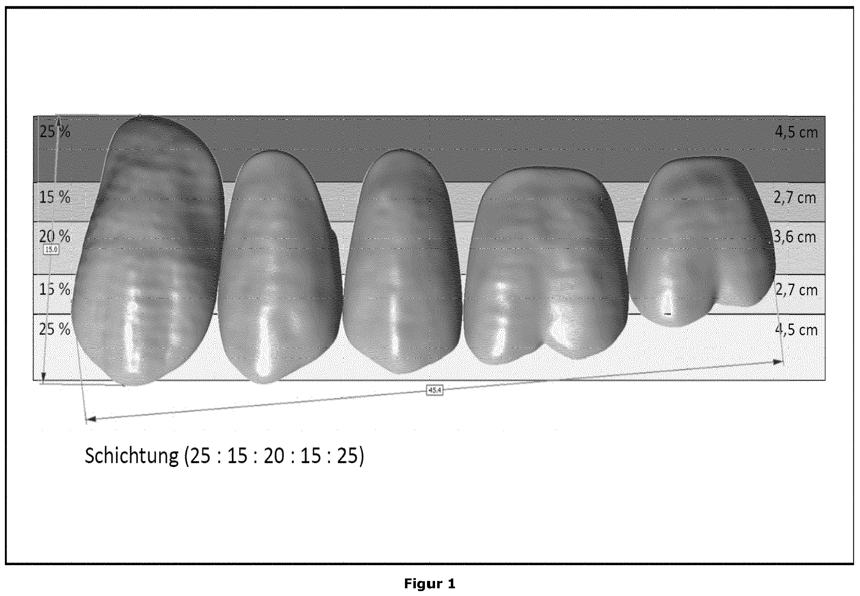

Figur 1 zeigt beispielhaft dentale Restaurationen, die aus dem beispielhaften keramischen Formkörper erhalten werden.

Die Schichtübergänge und Farbübergänge sind fließend. Die Restaurationen zeigen eine hervorragende Kantenfestigkeit und Stabilität auf. Ein Nacharbeiten und Nachjustieren der Zahnfarbe ist nicht erforderlich.

Der optimale Aufbau und die Zusammensetzung der Schichten zeigt eine über alle Schichten hinweg weitestgehende homogene Schwindung während der Sinterung. Dies ist insbesondere für eine passgenaue Anfertigung der dentalen Restaurationen von Vorteil, da aufwendiges Nacharbeiten weitestgehend vermieden werden kann.

## Patentansprüche

1. Verfahren zur Herstellung eines keramisches Formkörpers umfassend die folgenden Schritte:
a) Bereitstellen von 3 oder mehr keramischen Pulverschichten, die schichtweise übereinander angeordnet sind,
b) Verpressen der schichtweise übereinander angeordneten keramischen Pulverschichten zur Ausbildung eines Pressformkörpers und
c) Sintern des in Schritt b) erhaltenen Pressformkörpers zur Ausbildung eines keramischen Formkörpers, **dadurch gekennzeichnet, dass** die keramischen Pulverschichten jeweils eine voneinander verschiedene Zusammensetzung aufweisen, wobei jede keramische Pulverschicht eine Mischung aus mindestens 2 verschiedenen Basispulvern umfasst und die Basispulver jeweils mindestens 80 Gew.-% ZrO₂ und mindestens 0,02 Gew.-% Al₂O₃ aufweisen, wobei die Gewichtsangaben jeweils bezogen sind auf das Gesamtgewicht der Bestandteile des Basispulvers.

2. Verfahren gemäß Anspruch 1, **dadurch gekennzeichnet, dass** wenigstens eines, vorzugsweise wenigstens 2 oder wenigstens 3 der Basispulver, insbesondere alle Basispulver Y₂O₃ und/oder Er₂O₃, vorzugsweise in einer Menge von wenigstens 3 Gew.-%, insbesondere von wenigstens 5 Gew.-% oder wenigstens 6 Gew.-% und insbesondere von 4,5 bis 11 Gew.-%, speziell von 6 bis 10 Gew.-% bezogen auf das Gesamtgewicht der Bestandteile des Basispulvers, umfasst.

3. Verfahren gemäß Anspruch 1 oder 2, **dadurch gekennzeichnet, dass** wenigstens eines der Basispulver, vorzugsweise wenigstens 2 oder wenigstens 3 der Basispulver, färbende Metalloxide aufweist.

4. Verfahren gemäß einem oder mehreren der Ansprüche 1 bis 3, **dadurch gekennzeichnet, dass** wenigstens eines der Basispulver, vorzugsweise wenigstens 2 oder wenigstens 3 der Basispulver, Zirkondioxid und/oder HfO₂ in einer Menge von wenigstens 89 Gew.-%, vorzugsweise in einer Menge von 89 bis 98 Gew.-%, insbesondere von 90 bis 96 Gew.-%, jeweils bezogen auf das Gesamtgewicht der Bestandteile des Basispulvers, aufweist.

5. Verfahren gemäß einem oder mehreren der Ansprüche 1 bis 4, **dadurch gekennzeichnet, dass** jede keramische Pulverschicht mindestens 3 Basispulver, vorzugsweise 4 Basispulver, umfasst.

6. Verfahren gemäß einem oder mehreren der Ansprüche 1 bis 5, **dadurch gekennzeichnet, dass** 5 Pulverschichten bereitgestellt werden, die jeweils 4 voneinander unterschiedliche Basispulver in unterschiedlichen Mengen aufweisen.

7. Verfahren gemäß einem oder mehreren der Ansprüche 1 bis 6, **dadurch gekennzeichnet, dass** die Pulverschichten ein Basispulver A, enthaltend 92 bis 96 Gew.-% Zirkondioxid, 0,02 bis 0,1 Gew.-% Al₂O₃, 3,5 bis 6,5 Gew.-% Y₂O₃ und 0,02 bis 0,1 Gew.-Co₃O₄, wobei die Gewichtsangaben jeweils bezogen sind auf das Gesamtgewicht des Basispulvers A.

8. Verfahren gemäß einem oder mehreren der Ansprüche 1 bis 7, **dadurch gekennzeichnet, dass** die Pulverschichten ein Basispulver B, enthaltend 85 bis 93 Gew.-% Zirkondioxid, 0,02 bis 0,1 Gew.-% Al₂O₃ und 7,5 bis 11,0 Gew.-% Er₂O₃, wobei die Gewichtsangaben jeweils bezogen sind auf das Gesamtgewicht des Basispulvers B.

9. Verfahren gemäß einem oder mehreren der Ansprüche 1 bis 8, **dadurch gekennzeichnet, dass** die Pulverschichten ein Basispulver C, enthaltend 90 bis 94 Gew.-% Zirkondioxid, 0,02 bis 0,1 Gew.-% Al₂O₃ und 5,5 bis 8,0 Gew.-% Y₂O₃, wobei die Gewichtsangaben jeweils bezogen sind auf das Gesamtgewicht des Basispulvers C.

10. Verfahren gemäß einem oder mehreren der Ansprüche 1 bis 9, **dadurch gekennzeichnet, dass** die Pulverschichten ein Basispulver D, enthaltend 90 bis 94 Gew.-% Zirkondioxid, 0,02 bis 0,1 Gew.-% Al₂O₃, 5,5 bis 8,0 Gew.-% Y₂O₃ und 0,1 bis 0,3 Gew.-% Fe₂O₃, wobei die Gewichtsangaben jeweils bezogen sind auf das Gesamtgewicht des Basispulvers D.

11. Verfahren gemäß einem der vorangehenden Ansprüche, **dadurch gekennzeichnet, dass** sich wenigstens 2 der keramischen Pulverschichten hinsichtlich ihrer Dicke unterscheiden, vorzugsweise einen Dickenunterschied von wenigstens 5 % aufweisen.

12. Verfahren gemäß einem der vorangehenden Ansprüche, **dadurch gekennzeichnet, dass** wenigstens eine der äußeren keramischen Pulverschichten, vorzugsweise beide äußeren keramischen Pulverschichten eine größere Dicke aufweist/aufweisen als eine zwischen den äußeren keramische Pulverschichten liegende keramische Pulverschicht.

13. Verfahren gemäß einem oder mehreren der vorangehenden Ansprüche, **dadurch gekennzeichnet, dass** der in Schritt c) vorgesinterte keramische Formkörper durch subtraktive Verfahren bearbeitet wird und vorzugsweise anschließend in einem weiteren Schritt endgesintert wird.

14. Keramischer Formkörper erhältlich nach einem Verfahren gemäß einem der vorangehenden Ansprüche.

15. Verwendung des keramischen Formkörpers gemäß Anspruch 14 für dentale Restaurationen.
